# EUROPEAN PATENT APPLICATION

(11) **EP 4 257 147 A1**
(43) Date of publication of application: **11.10.2023**
(21) Application number: 21902941.0
(22) Date of filing: 01.09.2021
(51) Int. Cl.: A61K 45/00, A61K 31/192, A61P 27/10, A61P 43/00, C12Q 1/02, C12Q 1/68, G01N 33/15, G01N 33/50

(54) **EYEDROPS FOR INHIBITING MYOPIA PROGRESSION IN CHILDREN AND SCREENING METHOD FOR INHIBITOR OF MYOPIA PROGRESSION IN CHILDREN**

(30) Priority: 11.12.2020 JP 2020205489
(71) Applicant: Tsubota Laboratory, Inc., Tokyo 160-0016 (JP)
(72) Inventor: TSUBOTA Kazuo, Tokyo 160-0016 (JP); KURIHARA Toshihide, Tokyo 160-0016 (JP); IKEDA Shinichi, Tokyo 160-0016 (JP); MORI Kiwako, Tokyo 160-0016 (JP); JIANG Xiaoyan, Tokyo 160-0016 (JP)
(74) Representative: Mewburn Ellis LLP
(86) International application number: PCT/JP2021/032067
(87) International publication number: WO 2022/123836

(57) **Abstract**

The present invention provides a screening method for searching a component inhibiting the PERK pathway and/or the ATF6 pathway, and by obtaining, by the screening method, an active ingredient inhibiting myopia progression without impairing normal growth of the eyeballs (emmetropization) in children, provides eyedrops and a composition containing the active ingredient. The problems are solved by eyedrops for inhibiting myopia progression in children containing, as an active ingredient, an inhibitor of the PERK (PKR-like endoplasmic reticulum kinase) pathway and/or the ATF6 (activating transcription factor 6) pathway. The problems are solved by a screening method for an inhibitor of myopia progression in children, including a step of contacting a candidate substance with an eye-derived cell, and a step of selecting the candidate substance using, as an index, change in a protein and/or a gene of the signal transduction system of PERK and/or ATF6.

## Description

### Technical Field

The present invention relates to eyedrops used for inhibiting myopia progression in children, and a screening method for an inhibitor of myopia progression in children. More particularly, the present invention relates to an active ingredient capable of inhibiting only pathologic axial elongation causing myopia without inhibiting physiological axial elongation necessary for normal growth of the eyeballs in children ("normal growth of the eyeballs in children" being referred to as "emmetropization") by inhibiting ATF6 and/or PERK that are causal genes of myopia progression in children, and eyedrops containing the active ingredient, and a screening method for the active ingredient.

### Background Art

According to recent research on myopia and high myopia, the number of people with myopia is expected to remarkably increase worldwide, and the number of people with myopia is expected to be about five billion, and the number of people with high myopia is expected to be over about ten billion in 2050 (see Non Patent Literature 1).

Besides, according to epidemiological studies at Keio University School of Medicine in 2019, it has been revealed that prevalence of myopia in 689 elementary school students in Tokyo Prefecture is 76.5%, and in particular prevalence of myopia in students in first grade is already over 60%. Similarly, prevalence of myopia in 727 junior high school students in Tokyo Prefecture is 94.9%, and is over 90% in all the grades (see Non Patent Literature 2). This prevalence is higher than those reported in other East Asian countries, and it can be said that myopia in children in Japan has reached a very serious level.

Eyes of a human are hyperopic immediately after birth, and thereafter, since the optical axis elongates owing to axial elongation in the front-back direction in the growth period (up to 8 years old), the degree of hyperopia is reduced, and the eyes become emmetropic in the school period when a suitable axial length is obtained. This is designated as "physiological axial elongation", and when this physiological axial elongation is impaired for some reason, the axial elongation becomes insufficient and hence hyperopia remains, which remarkably reduces QOL (quality of life) of the child. On the other hand, a state where the axial elongation does not stop even after emmetropization and the axial length excessively elongates after 8 years old is designated as "pathologic axial elongation", and this is a cause of myopia progression in children, and therefore, myopia abruptly progresses in the school period of 8-year-old or older ages, and the axial length once elongated cannot be restored (see Non Patent Literature 3).

In order to inhibit myopia progression in children, it is necessary to inhibit this excessive axial elongation (pathologic axial elongation), but if physiological axial elongation is also inhibited, the eye does not become myopic but remains hyperopic, which defeats the purpose. Therefore, in appropriate strategy for inhibiting myopia progression in children, it is necessary to simultaneously attain conflicting effects of inhibiting pathologic axial elongation and of not inhibiting physiological axial elongation simultaneously.

### Citation List

### Patent Literature

Patent Literature 1: International Publication No. WO2018/164113

### Non Patent Literature

Non Patent Literature 1: Brien A Holden, et.al., "Global prevalence of myopia and high myopia and temporal trends from 2000 through 2050", Ophthalmology, Vol. 123, Number 5, P.1036-1042 (May 2016).
Non Patent Literature 2: "Myopia on the Rise Among Elementary and Junior High School Students", Keio University School of Medicine, Press Release, August 19, 2019 (https://oklens.co.jp/new/2019/08/21/).
Non Patent Literature 3: Takashi Fujikado, "Nihon Ganka Gakkai Senmon-i Seido Shogai Kyouiku Koza (Japanese Ophthalmological Society Board Certification System Lifelong Education Course) Review 54, Shoni no Kinshi no Shinko Boshi (Prevention of Progression of Child Myopia), Japanese Journal of Ophthalmology, vol. 117, No. 4, pp. 397-406 (April 10, 2013).
Non Patent Literature 4: Jiang, X., et.al., A highly efficient murine model of experimental myopia, Scientific reports 8, 2026, doi: 10.1038/s 41598-018-20272-w (2018).
Non Patent Literature 5: Mori, K., et.al., Oral crocetin administration suppressed refractive shift and axial elongation in a murine model of lens-induced myopia, Scientific reports 9, 295, doi: 10.1038/s41598-018-36576-w (2019).
Non Patent Literature 6: Xiangtian Zbou, et al., The Development of the Refractive Status and Ocular Growth in C57BL6 Mice, Investigative Ophthalmology & Visual Science, December 2008, Vol.49, No. 12.

### Summary of Invention

### Technical Problem

In recent years, causative factors of myopia progression in children (pathologic axial elongation) have been clarified. The present inventors have earnestly studied this group of factors, resulting in finding that genes responding unfolded protein (endoplasmic reticulum stress response genes), that is, an abnormal protein in the endoplasmic reticulum, are strongly involved in pathologic axial elongation (see Patent Literature 1). As this group of genes, three genes of PERK (PKR-like endoplasmic reticulum kinase), ATF6 (activating transcription factor 6), and IRE1 (inositol requiring 1) are known, but detailed contribution rates of the pathways, for example, which gene pathway should be inhibited, and whether or not all the pathways may be inhibited, have been unknown. When the inhibition of these gene pathways is insufficient, pathologic axial elongation cannot be expected to be sufficiently inhibited, and alternatively, when the pathways are excessively inhibited, also physiological axial elongation may be inhibited, and therefore, it has been considered that inhibition of myopia progression in children where pathologic axial elongation and physiological axial elongation simultaneously occur is extremely difficult.

### Solution to Problem

The present inventors have studied the degree of involvement, in pathologic axial elongation, of the signal transduction systems (pathways) of the endoplasmic reticulum stress response genes (PERK, ATF6, and IRE1), the contribution rates of the inhibition of the respective pathways to axial elongation inhibition, and synergic effects obtained in inhibition by a combination of these. As a result, it has been found that the effect of inhibiting pathologic axial elongation is increased by inhibiting the PERK pathway and/or the ATF6 pathway. Besides, it has been found that it is significant for inhibition of myopia progression in children to selectively inhibit at least the ATF6 pathway, and that when both the PERK pathway and the ATF6 pathway are inhibited, pathologic axial elongation (myopia progression) in children is sufficiently inhibited but physiological axial elongation (emmetropization) is not inhibited.

An object of the present invention is to provide a screening method for searching a component inhibiting the PERK pathway and/or the ATF6 pathway. Another object is to obtain, by the screening method, an active ingredient inhibiting myopia progression without impairing normal growth of the eyeballs (emmetropization) in children to provide eyedrops containing the active ingredient. In addition, another object is to provide a method for verifying whether or not each of various components considered effective for myopia is safe for children in which pathologic axial elongation and physiological axial elongation simultaneously progress, namely, whether or not it causes hyperopia due to insufficient elongation or myopia due to excessive elongation.

Specifically, the present invention provides the following:
[1] Eyedrops for inhibiting myopia progression in children, comprising an inhibitor of PERK (PKR-like endoplasmic reticulum kinase) pathway and/or ATF6 (activating transcription factor 6) pathway as an active ingredient.
[2] The eyedrops according to [1] described above, wherein the inhibitor is at least one selected from the group consisting of phenylbutyric acid and pharmacologically acceptable salts thereof.
[3] The eyedrops according to [1] or [2] described above, wherein the inhibitor is sodium phenylbutyrate.
[4] The eyedrops according to any one of [1] to [3] described above, wherein a content of the inhibitor is 0.01 to 5% by mass based on a total amount of the eyedrops.
[5] The eyedrops according to any one of [1] to [4] described above, in which inhibition of the myopia progression in children does not inhibit physiological axial elongation.
[6] The eyedrops according to any one of [1] to [5] described above, in which inhibition of the myopia progression in children inhibits pathologic axial elongation.
[7] A screening method for an inhibitor of myopia progression in children, comprising a step of contacting a candidate substance with an eye-derived cell; and a step of selecting the candidate substance by using, as an indicator, change in a protein and/or a gene of a signal transduction system of PERK and/or ATF6 in the cell.

### Advantageous Effects of Invention

The present invention can provide a screening method for searching a component inhibiting the signal transduction systems of PERK and/or ATF6. Owing to this screening method, an active ingredient inhibiting myopia progression without impairing physiological axial elongation (emmetropization) in children can be provided, and thus, eyedrops and a composition containing the active ingredient can be provided.

### Brief Description of Drawings

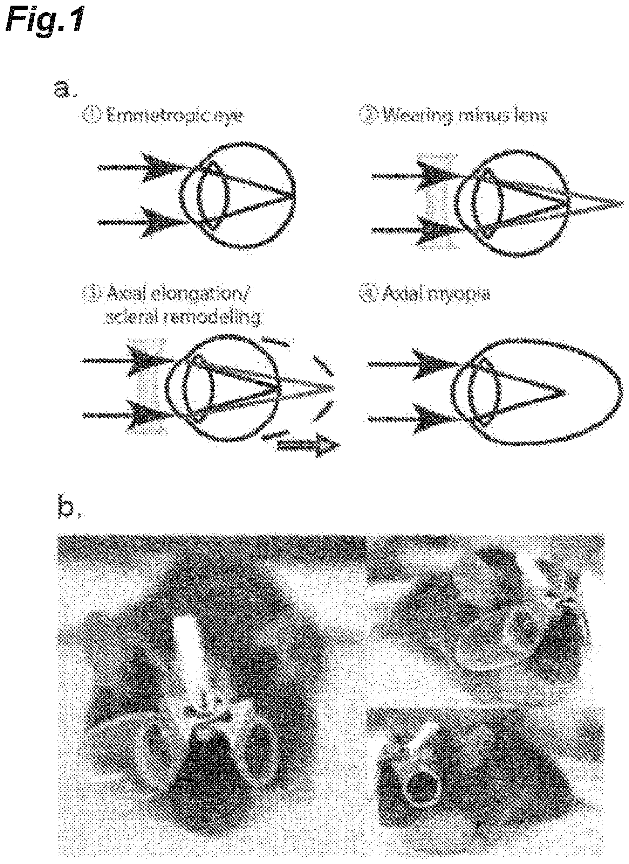

[Figure 1] Figure 1 is an explanatory diagram of myopia induction in a juvenile mouse. Figure 1(a) illustrates schematical structural views of the myopia induction, and Figure 1(b) illustrates photographs of myopia induced juvenile mice.

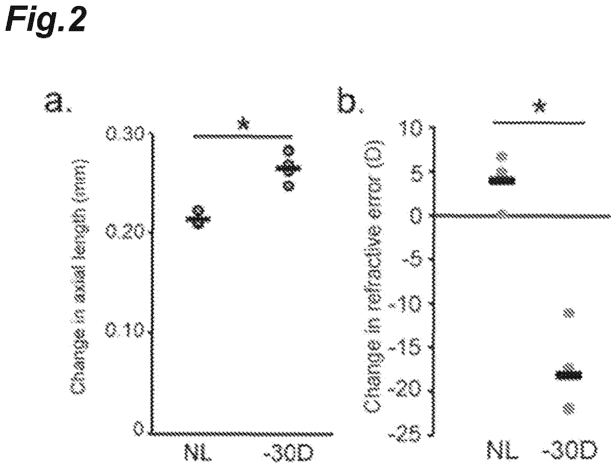

[Figure 2] Figure 2 is a graph illustrating that myopia induction induces axial elongation and endoplasmic reticulum stress in the sclera. Figure 2(a) illustrates change in axial length obtained by myopia induction for 3 weeks in mice (n = 4) (*p < 0.05), and Figure 2(b) illustrates refraction change obtained by myopia induction for 3 weeks in mice (n = 4) (*p < 0.05).

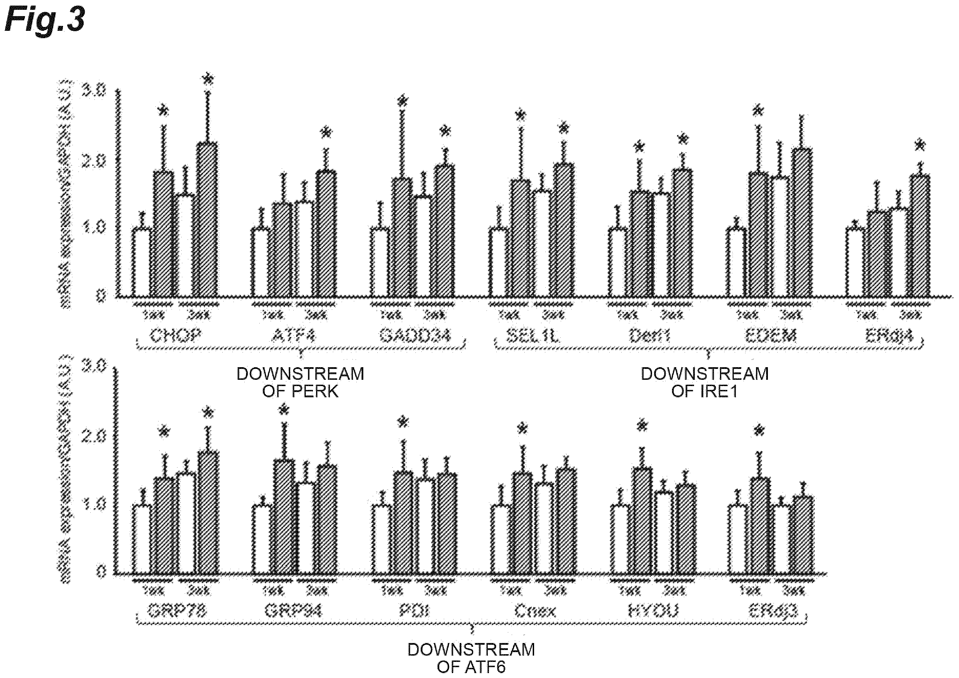

[Figure 3] Figure 3 illustrates the expression levels of endoplasmic reticulum stress response genes measured by quantitative PCR in 1st week and 3rd week of myopia induction in the sclera of control eyes (white columns) and myopia-induced eyes (gray columns) of mice (n = 8 in each group) (*p < 0.05).

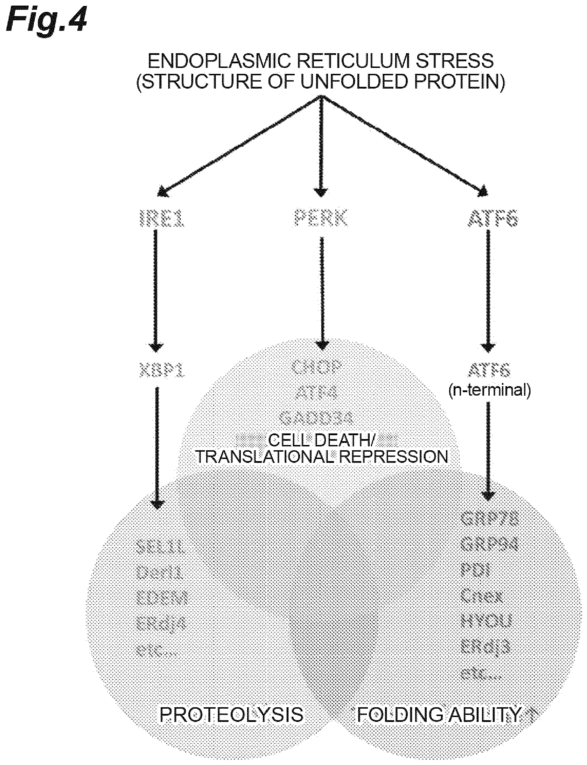

[Figure 4] Figure 4 is an explanatory diagram of the PERK pathway, the ATF6 pathway, and the IRE1 pathway, that is, endoplasmic reticulum stress response genes.

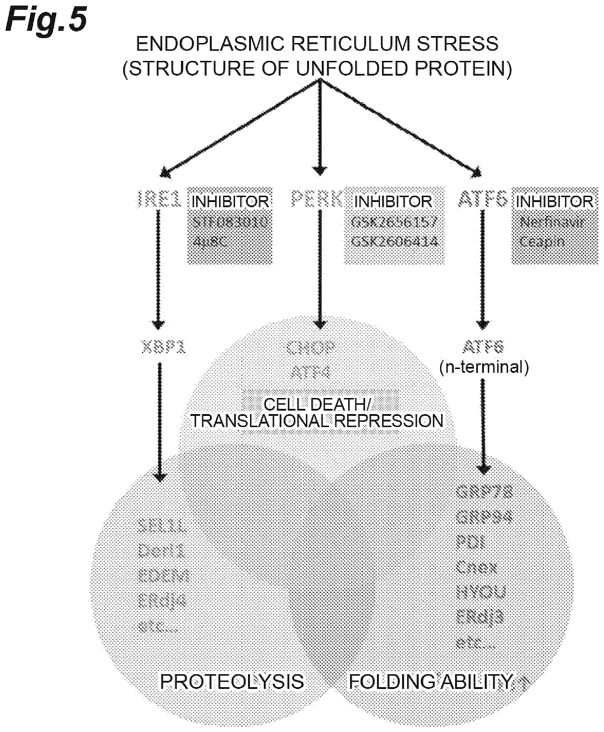

[Figure 5] Figure 5 is an explanatory diagram of various inhibitors of the PERK pathway, the ATF6 pathway, and the IRE1 pathway, that is, endoplasmic reticulum stress response genes.

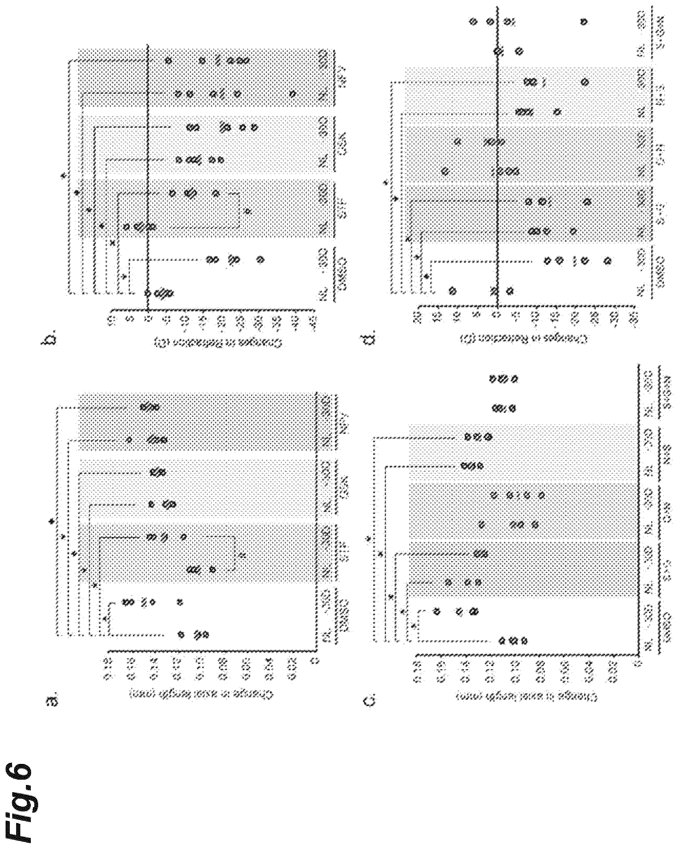

[Figure 6] Figure 6 illustrates graphs indicating axial elongation and refraction change (myopia) caused in mice by eyedrop of various inhibitors for the PERK pathway, the ATF6 pathway and the IRE1 pathway. Figure 6(a) is a graph illustrating influence on the axial elongation caused by eyedrop administration of single one of STF080310 (STF), GSK2656157 (GSK) and nelfinavir (NEV) (n = 5 in each group), and illustrating results of comparison with a DMSO eye-dropped NL (= no lens) group (*p < 0.05), and results of comparison with a STF eye-dropped NL group or a -30 D lens wearing group (#p < 0.05). Figure 6(b) illustrates results of influence on myopic refraction caused by eyedrop administration of single one of STF, GSK and NFV (n = 5 in each group), and illustrates results of comparison with a DMSO eye-dropped NL group (*p < 0.05), and results of comparison with a STF eye-dropped NL group or a -30 D lens wearing group (#p < 0.05). Figure 6(c) illustrates results of influence on the axial elongation caused by eyedrop administration of STF, GSK and NFV in combination (n = 4 in each group), and illustrates results of comparison with a DMSO eye-dropped NL group (*p < 0.05). Figure 6(d) illustrates results of influence on myopic refraction caused by eyedrop administration of STF, GSK and NFV in combination (n = 4 in each group), and illustrates results of comparison with a DMSO eye-dropped NL group (*p < 0.05).

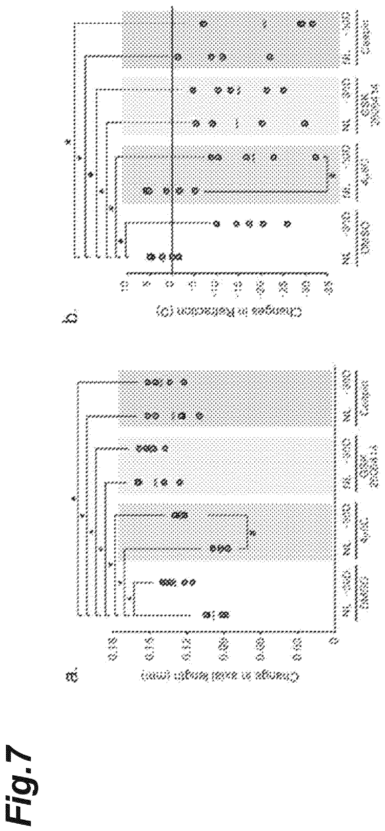

[Figure 7] Figure 7 illustrates graphs indicating axial elongation and refraction change (myopia) caused in mice by eyedrop administration of various inhibitors, different from those of Figure 6, for the PERK pathway, the ATF6 pathway and the IRE1 pathway. Figure 7(a) illustrates graphs of influence on axial elongation caused by eyedrop administration of single one of 4µ8C, GSK2606414, and Ceapin-A7 (n = 5 in each group), and illustrates results of comparison with a DMSO eye-dropped NL (= no lens) group (*p < 0.05), and results of comparison with a 4µ8C eye-dropped NL group or a -30 D lens wearing group (#p < 0.05). Figure 7(b) illustrates influence on myopic refraction caused by eyedrop administration of single one of 4µ8C, GSK2606414, and Ceapin-A7 (n = 5 in each group), and illustrates results of comparison with a DMSO eye-dropped NL (= no lens) group (*p < 0.05), and results of comparison with a 4µ8C eye-dropped NL group or a -30 D lens wearing group (#p < 0.05).

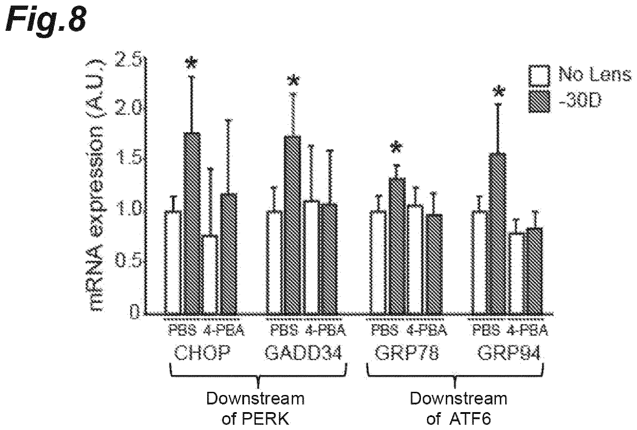

[Figure 8] Figure 8 is a graph illustrating that myopia induction in a mouse induces endoplasmic reticulum stress in the sclera, wherein the expression of endoplasmic reticulum stress response genes is measured by quantitative PCR in the sclera (n = 6 in each group) having PBS intraperitoneally injected (PBS) or having sodium phenylbutyrate administered (4-PBA; 200 mg/kg/day), for showing results that the gene expression is increased in myopia induced eyes (gray columns) as compared with in control eyes (white columns), and that the increase is inhibited by 4-PBA (*p < 0.05).

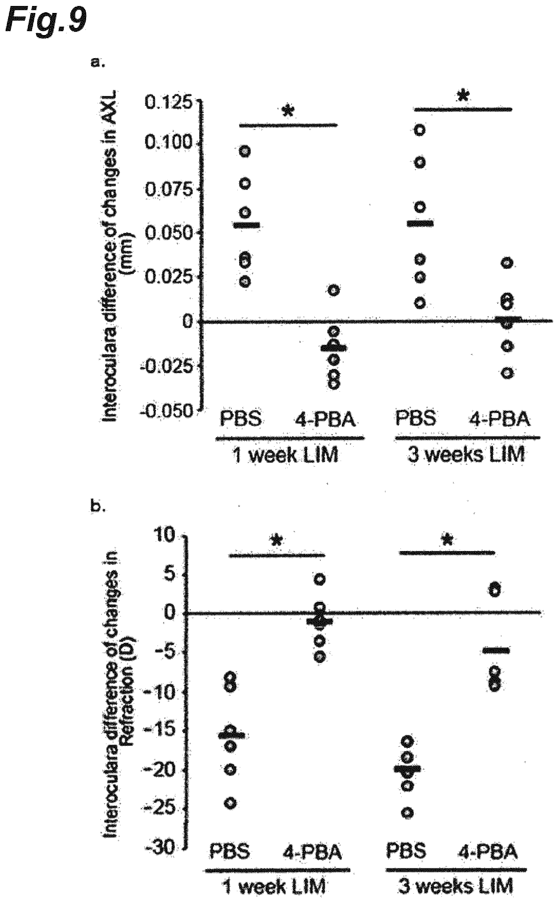

[Figure 9] Figure 9 is a graph illustrating that myopia induction in a mouse induces axial elongation and myopic refraction. Figure 9(a) illustrates results indicating that the axial elongation is inhibited by intraperitoneal injection of phenylbutyric acid in 1st week and 3rd week of the myopia induction (LIM) (4-PBA; 200 mg/kg/day) (n = 6 in each group, *p < 0.05), and Figure 9(b) illustrates results indicating that the myopic refraction is inhibited by 4-PBA administration in 1st week and 3rd week of the myopia induction (LIM) (n = 6 in each group, *p < 0.05).

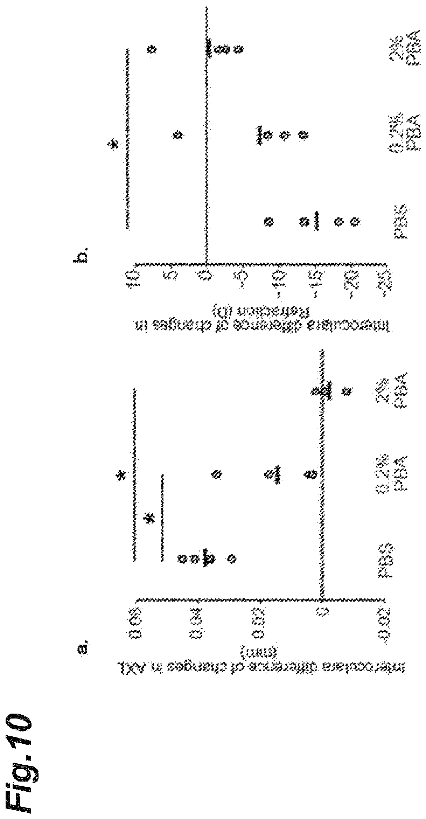

[Figure 10] Figure 10 is a graph illustrating that myopia induction in a mouse induces axial elongation and myopic refraction. Figure 10(a) is a graph illustrating that eyedrop administration of 4-PBA inhibits axial elongation dose-dependently (n = 4 in each group, *p < 0.05), and Figure 10(b) is a graph illustrating that eyedrop administration of 4-PBA inhibits myopic refraction dose-dependently (n = 4 in each group, *p < 0.05).

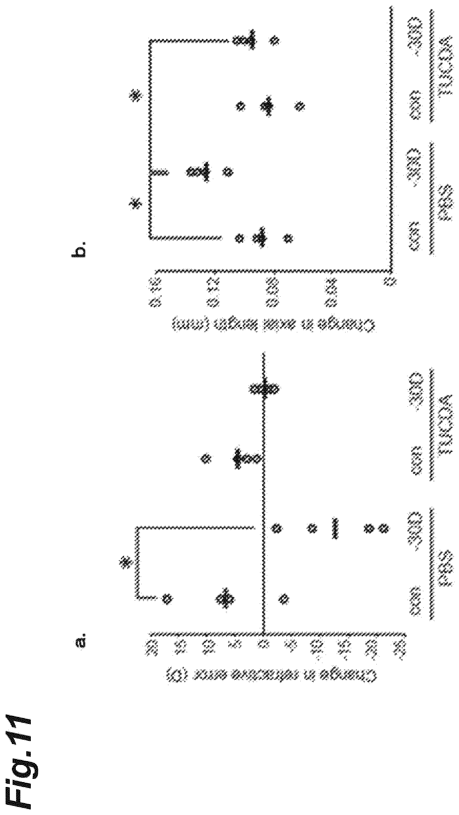

[Figure 11] Figure 11 is a graph illustrating that myopia induction in a mouse induces axial elongation and myopic refraction. Figure 11(a) is a graph illustrating that myopic refraction is inhibited by intraperitoneal injection of tauroursodeoxycholic acid (TUDCA; 100 mg/kg) dose-dependently (n = 4 in each group, *p < 0.05), and Figure 10(b) is a graph illustrating that axial elongation is inhibited by TUDCA (n = 4 in each group, *p < 0.05).

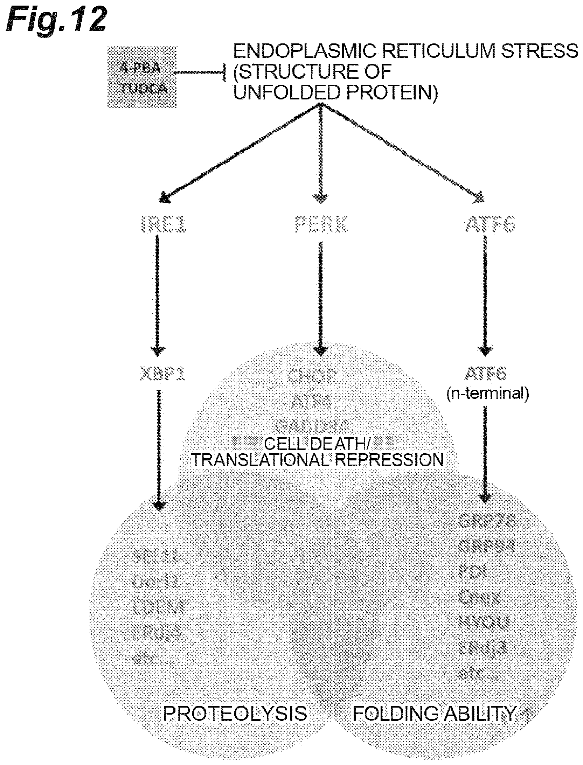

[Figure 12] Figure 12 is an explanatory diagram illustrating mechanism of inhibiting, by 4-PBA and TUDCA, the PERK pathway, the ATF6 pathway, and the IRE1 pathway, that is, the endoplasmic reticulum stress response genes.

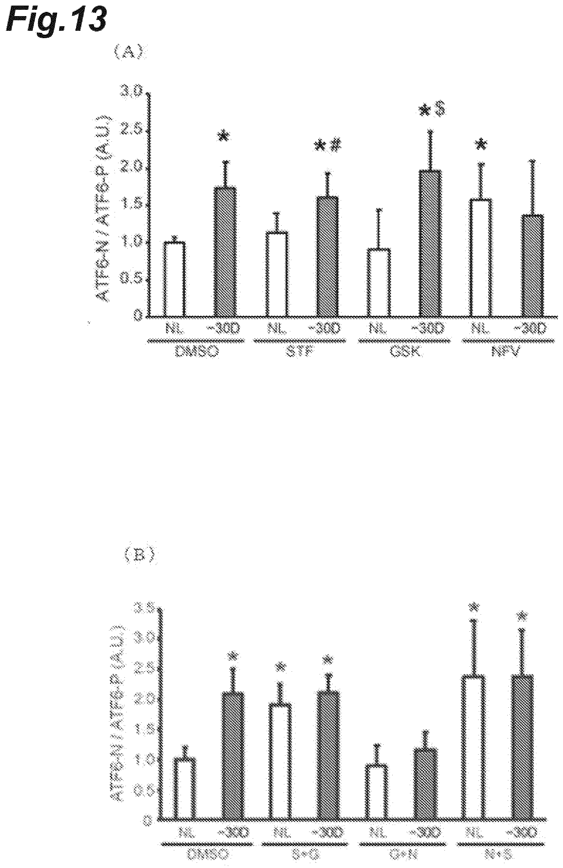

[Figure 13] Figure 13 is a graph illustrating evaluation of involvement of the ATF6 pathway in inhibition of myopia progression in children obtained in Test Example 4.

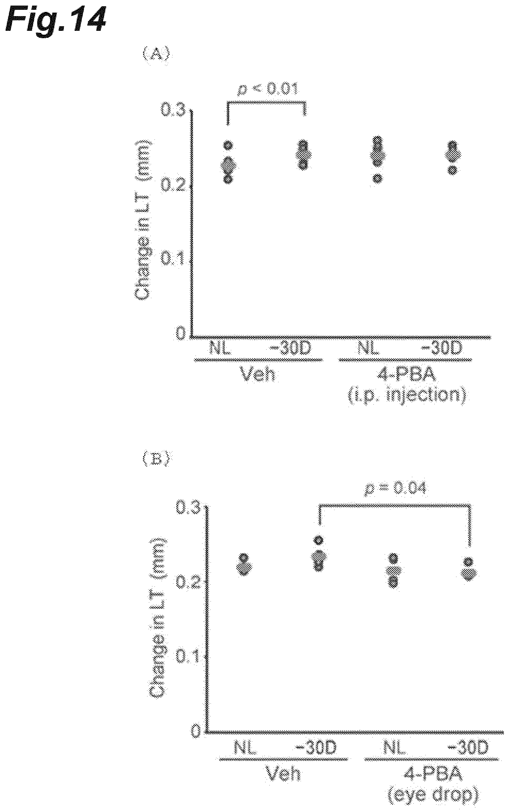

[Figure 14] Figure 14 illustrates graphs, obtained in Test Example 5, of influence of myopia induction on lens thickening depending on a difference in dosage form.

### Description of Embodiments

The present invention will now be described in detail. The present invention is not limited to the following embodiments and experimental examples but encompasses various modification examples and application examples within the scope of the present invention.

### [Eyedrops for Inhibiting Myopia Progression in Children]

Eyedrops for inhibiting myopia progression in children of the present invention contains, as an active ingredient, an inhibitor of the PERK (PKR-like endoplasmic reticulum kinase) pathway and/or the ATF6 (activating transcription factor 6) pathway.

Herein, the inhibitor of the PERK pathway and/or the ATF6 pathway refers to a substance having an inhibitory effect on the signal transduction system of PERK (the PERK pathway) and/or the signal transduction system of ATF6 (the ATF6 pathway). The effect of inhibiting these signal transduction systems can be evaluated, as described in Examples below, by a known method using, as an index, change in a gene and/or a protein involved in these signal transduction systems.

### (Inhibitor of PERK Pathway and/or ATF6 Pathway)

As described above, as a factor for myopia progression in children (pathologic axial elongation), a gene pathway responding to an unfolded protein that is an abnormal protein in the endoplasmic reticulum is involved in the pathologic axial elongation. As the gene pathway, three pathways of the PERK pathway, the ATF6 pathway, and the IRE1 pathway are known, and as described in experimental examples below, it has been newly found that it is indispensable for myopia inhibition to inhibit at least the ATF6 pathway. It has been also newly found that the effect of inhibiting myopia progression in children is further increased by inhibiting the PERK pathway and the ATF6 pathway among these three pathways. It has been also confirmed that when only one of the PERK pathway and the ATF6 pathway is inhibited, the other pathway may be activated in compensation. Therefore, in one embodiment, although not limited, a substance having an inhibitory effect on both the PERK pathway and the ATF6 pathway can be an active ingredient for inhibiting myopia progression in children.

In other words, a compound that targets and reduces a gene or a protein involved in signal transduction of PERK and/or ATF6, or a nucleic acid such as antisense oligonucleotide or siRNA that reduces protein expression in the PERK pathway and/or the ATF6 pathway can be blended in eyedrops as an ingredient effective for inhibiting myopia progression in children.

Herein, the inhibitor of the PERK pathway or the ATF6 pathway refers to a substance having an inhibitory effect on the signal transduction system of PERK or the signal transduction system of ATF6 in the endoplasmic reticulum. The inhibitory effect on these signal transduction systems can be evaluated by using, as an indicator, change in a gene and/or a protein involved in these signal transduction systems by a method described in experimental examples below, or by a known method.

In evaluation of expression of a gene or expression of a protein of a factor involved in the signal transduction system of PERK, the evaluation can be performed in accordance with that the expression of the factor is varied by at least 1% by a candidate substance as compared with a control not having the candidate substance added thereto.

Besides, in evaluation of expression of a gene or expression of a protein of a factor involved in the signal transduction system of ATF6, the evaluation can be performed in accordance with that the expression of the factor is varied by at least 1% by a candidate substance as compared with a control not having the candidate substance added thereto.

PERK is endoplasmic reticulum transmembrane kinase, and examples of the factor involved in the signal transduction include eIF2a (eukaryotic initiation factor 2α), ATF4 (activating transcription factor 4), CHOP (C/EBP homologous protein), and GADD34 (growth arrest DNA and damage protein 34).

Besides, ATF6 is a membrane-bound transcription factor belonging to the CREB/ATF family, and examples of the factor involved in the signal transduction include BiP (binding immunoglobulin protein, also referred to as "GRP78"), Txndc12 (thioredoxin domain containing 12, also referred to as "ERp18"), S1P (site-1 protease), and S2P (site-2 protease).

In experimental examples described below, at least one selected from the group consisting of phenylbutyric acid, tauroursodeoxycholic acid, and pharmacologically acceptable salts thereof has been found by screening a component capable of inhibiting both the PERK pathway and the ATF6 pathway. The component is, however, not limited to this, but a component newly specified as a component inhibiting at least the ATF6 pathway, and a component newly specified as a component inhibiting the PERK pathway and the ATF6 pathway can be used. The inhibitor of the ATF6 pathway is not limited, and from the viewpoint of solubility in eyedrops, sodium phenylbutyrate is preferred. As described in experimental examples below, sodium phenylbutyrate is preferred because not only the ATF6 pathway but also the PERK pathway can be inhibited. The inhibitor of the PERK pathway and/or the ATF6 pathway may be synthesized by a known method to be used, or a commercially available product may be obtained to be used.

Herein, the "pharmaceutically acceptable salt" is not especially limited, and specific examples include organic acid salts, inorganic acid salts, organic base salts, and inorganic base salts. Examples of the organic acid salts include monocarboxylic acid salts such as acetic acid salt, trifluoroacetic acid salt, butyric acid salt, palmitic acid salt, and stearic acid salt; polycarboxylic acid salts such as fumaric acid salt, maleic acid salt, succinic acid salt, and malonic acid salt; oxycarboxylic acid salts such as lactic acid salt, tartaric acid salt, and citric acid salt; and organic sulfonic acid salts such as methanesulfonic acid salt, toluenesulfonic acid salt, and tosic acid salt. Examples of the inorganic acid salts include hydrochloric acid salt, sulfuric acid salt, nitric acid salt, hydrobromic acid salt, and phosphoric acid salt. Examples of a salt with an organic base include salts with organic amines such as methyl amine, triethyl amine, triethanol amine, diethanol amine, morpholine, piperazine, pyrrolidine, tripyridine, picoline, and ethylene diamine. Examples of a salt with an inorganic base include various salts such as ammonium salt; and salts with alkali metals such as sodium and potassium, alkaline earth metals such as calcium and magnesium, and metals such as aluminum. One of these salts may be singly used, or two or more of these may be used in an optional combination. The "pharmaceutically acceptable salt" may include a solvate or a hydrate of a salt.

A content of the inhibitor of the PERK pathway and/or the ATF6 pathway can be appropriately changed depending on an administration method, a dosage, the type of additive and the like. For example, the content is preferably 0.01% by mass or more, more preferably 0.05% by mass or more, further preferably 0.1% by mass or more, and particularly preferably 0.2% by mass or more based on the total amount of the eyedrops (corresponding to a total mass; the same applies herein). Besides, the content of the inhibitor of the PERK pathway and/or the ATF6 pathway is, for example, preferably 5% by mass or less, more preferably 4% by mass or less, further preferably 3% by mass or less, and particularly preferably 2% by mass or less based on the total amount of the eyedrops. Besides, the content of the inhibitor of the PERK pathway and/or the ATF6 pathway is, for example, preferably 0.01 to 5% by mass, more preferably 0.05 to 4% by mass, further preferably 0.1 to 3% by mass, and particularly preferably 0.2 to 2% by mass based on the total amount of the eyedrops.

When at least one selected from the group consisting of phenylbutyric acid and pharmacologically acceptable salts thereof is used as the inhibitor of the PERK pathway and/or the ATF6 pathway, the content is, for example, preferably 0.01 to 5% by mass, more preferably 0.05 to 4% by mass, further preferably 0.1 to 3% by mass, and particularly preferably 0.2 to 2% by mass based on the total amount of the eyedrops.

### [Usage]

The eyedrops of the present invention are used for inhibiting myopia progression in children. Herein, a child refers to a child of 7 years old or older, and younger than 15 years old. As for the degree of refraction of the eye, the eye is slightly hyperopic after birth, and the eye axis elongates and the eye becomes substantially emmetropic before reaching the school age.

The axial length rapidly elongates after birth up to about 2 years old, and thereafter gradually elongates. Such axial elongation along with growth until emmetropization is designated as "physiological axial elongation", and is an indispensable phenomenon for normal growth of the eye. Continuous elongation of the axial length even after school age or older leads, however, to progression of myopia, and hence is regarded as "pathologic axial elongation". For example, in pathologic axial elongation, elongation of the axial length by 1 mm in an adult eye leads to increase of the degree of myopia by about 3.0 D, and the axial elongation does not restore.

Therefore, in inhibition of myopia progression in children, it is required to inhibit pathologic axial elongation (myopia progression) without inhibiting physiological axial elongation (emmetropization). In experimental examples described below, it has been newly found that it is indispensable for myopia inhibition to inhibit at least the ATF6 pathway. It has been also confirmed that when either one of the PERK pathway and the ATF6 pathway alone is inhibited, the other pathway may be activated in compensation in some cases. Accordingly, although not limited, it has been found that the effect of inhibiting pathologic axial elongation is synergistically increased by inhibiting both the PERK pathway and the ATF6 pathway in one embodiment. Owing to this mechanism, myopia progression in children can be inhibited without inhibiting physiological axial elongation (emmetropization).

### (Dosage Form)

A composition of the present invention is used in the form of eyedrops. In the present invention, the dosage form of the eyedrops for inhibiting myopia progression in children is not limited, and examples include aqueous eyedrops, eyedrops dissolved before use, suspension eyedrops, oily eyedrops, and an eye ointment. Among these, the dosage form is preferably aqueous eyedrops from the viewpoint of remarkably exhibiting the effects of the present invention.

In the eyedrops, other active ingredients (such as a pharmacologically active ingredient, and a physiological active ingredient) can be blended in addition to the above-described component. The type of such an ingredient is not especially limited, and examples include a decongestant component, an eye muscle adjusting agent component, an anti-inflammatory agent component, an astringent component, an antihistamine component, an antiallergic agent component, vitamins, amino acids, an antimicrobial component, sugars, polymer compounds or derivatives thereof, cellulose or derivatives thereof, and a local anesthetic component.

The eyedrops can further contain one, two or more of various components and additives appropriately selected by a conventional method in accordance with the usage and form as long as the effects of the present invention are not impaired. Examples of these components and additives include various additives such as a carrier generally used in preparation of a liquid medicine, a perfume or cooling agent, a preservative, a bactericide or antibacterial agent, a pH adjuster, a chelating agent, a stabilizer, a tonicity agent, a buffer, and a thickening agent. Examples of representative components used in eyedrops include, but are not limited to, the following.

Examples of the carrier include water, and an aqueous solvent such as hydrous ethanol. When various components are difficult to be dissolved in an aqueous solvent, a solubilizing agent may be used. Examples of the solubilizing agent include polyoxyethylene hydrogenated castor oil, polyoxyl 40 stearate, povidone, and polysorbate 80.

Examples of the perfume or cooling agent include terpenes (specifically such as anethol, eugenol, camphor, geraniol, cineole, borneol, menthol, limonene, and borneo camphor; all of which may be in any of d-form, l-form, and dl-form), and essential oils (such as fennel oil, cool mint oil, cinnamon oil, spearmint oil, peppermint water, mint oil, peppermint oil, bergamot oil, eucalyptus oil, and rose oil).

Examples of the preservative, and the bactericide or antibacterial agent include polidronium chloride, alkyldiaminoethylglycine hydrochloride, sodium benzoate, ethanol, benzalkonium chloride, benzethonium chloride, chlorhexidine gluconate, chlorobutanol, sorbic acid, potassium sorbate, sodium dehydroacetate, methyl parahydroxybenzoate, ethyl parahydroxybenzoate, propyl parahydroxybenzoate, butyl parahydroxybenzoate, oxyquinoline sulfate, phenethyl alcohol, benzyl alcohol, biguanide compounds (specifically such as polyhexamethylene biguanide, and hydrochlorides thereof), and Glow Kill (name of a product manufactured by Rhodia).

Examples of the pH adjuster include hydrochloric acid, sodium hydroxide, potassium hydroxide, calcium hydroxide, magnesium hydroxide, triethanolamine, monoethanolamine, diisopropanolamine, sulfuric acid, and phosphoric acid.

Examples of the chelating agent include ascorbic acid, tetrasodium edetate, sodium edetate, and citric acid.

Examples of the stabilizer include sodium edetate hydrate, povidone, polysorbate 80, dibutylhydroxytoluene, trometamol, sodium formaldehyde sulfoxylate (rongalite), tocopherol, sodium metabisulfite, monoethanolamine, aluminum monostearate, and glycerin monostearate.

Examples of the tonicity agent include potassium chloride, sodium chloride concentrated glycerin, glucose, and D-mannitol.

Examples of the buffer include sodium citrate hydrate, sodium acetate hydrate, sodium bicarbonate, trometamol, boric acid, borax, sodium hydrogen phosphate hydrate, and sodium dihydrogen phosphate.

Examples of the thickener include a carboxyvinyl polymer, povidone, polyvinyl alcohol (partially saponified), hydroxyethylcellulose, hypromellose, methylcellulose, and glycerin.

In the eyedrops of the present invention, these additives can be blended in expectation of the effects of the present invention, or as long as the effects of the present invention are not impaired. The content is not especially limited, and is preferably about 0.001 to 1% by mass based on the total amount of the eye drops.

The pH of the eyedrops may be 3 to 10, and is preferably 4 to 9 from the viewpoint of usability, and more preferably 5 to 8.5 from the viewpoint of usability.

As a container for holding the eyedrops of the present invention, any of known eyedroppers can be used without limitation. As an eyedropper, any container having a shape capable of dropping eyedrops onto an eye, for example, a shape having a nozzle and a container mouth at the tip of the nozzle can be used. Besides, the eyedropper may be either of one having a structure in which a separately molded nozzle is attached onto a container, and one having a structure in which a nozzle portion (liquid injection portion) and a container body are integrally molded (such as a disposable eyedropper).

The eyedropper may be usually a plastic container. The constituent material of the plastic container is not especially limited, and examples include one of polyethylene terephthalate, polyarylate, polyethylene naphthalate, polycarbonate, polyethylene, polypropylene, and polyimide, a copolymer of these, and a mixture of two or more of these. From the viewpoint that the effects of the present invention are easily exhibited depending on the extent of pushing out, polyethylene terephthalate, polyarylate, polyethylene naphthalate, a copolymer of these, or a mixture of two or more of these are particularly preferred.

The eyedrops may be filled in a transparent container (a container having transparency sufficient for observing a foreign matter therein) using such a material as a principal material, or may be filled in a light resistant container. The light resistance may be obtained, for example, by adding a colorant to a transparent container material, or may be obtained by covering the container with a shrink film, an outer case or the like. Besides, the volume of the container is preferably about 0.5 to 50 mL, and more preferably about 3 to 20 mL for more easily exhibiting the effects of the present invention depending on the extent of pushing out.

Besides, the nozzle provided on the eyedropper is also not especially limited in the structure and the constituent material. The structure of the nozzle may be any structure generally employed as the nozzle of an eyedropper. Besides, examples of the constituent material of the nozzle are similar to those described above regarding the constituent material of the plastic container. From the viewpoint of making more favorable the anti-drip property of the eyedrops, and suppressing variation in drop amount, a nozzle containing polyethylene or polypropylene as the constituent material is favorable. Examples of the type of polyethylene include high density polyethylene and low density polyethylene, and in particular, a nozzle containing low density polyethylene as the constituent material is favorable.

### (Method for Producing Eyedrops)

The eyedrops of the present invention can be prepared by a method commonly employed by or known to those skilled in the art. For example, the preparation may be performed by dispersing respective components in a carrier such as water, adding a solubilizing agent thereto if necessary, heating the resultant if necessary, homogenizing, dissolving or emulsifying the resultant with a homomixer or the like, and adjusting the pH with a pH adjuster. Besides, as a method for sterilizing the formulation, electron beam sterilization, autoclave sterilization, filtration sterilization or the like can be selected.

### (Usage)

The administration method and the dosage of the eyedrops of the present invention are varied depending on the symptom of a patient and the like, and about 1 to 2 drops each may be usually eye-dropped about once to 6 times a day.

The eyedrops of the present invention can be applied to a child. Although not limited, when eyedrops containing at least one selected from the group consisting of phenylbutyric acid and pharmacologically acceptable salts thereof are used as the inhibitor of the PERK pathway and/or the ATF6 pathway, for example, 1 to 2 drops of the eyedrops can be eye-dropped each once or twice a day, and it is preferable to eye-drop 1 drop each once a day.

Besides, from the viewpoint of remarkably exhibiting the effects of the present invention, the eyedrops of the present invention can be used in a child, for example, in an inactive time period, for example, before a nap, before bedtime or the like.

### [Screening Method for Inhibitor of Myopia Progression in Children]

In the present invention, a screening method for an inhibitor of myopia progression in children includes a step of contacting a candidate substance with an eye-derived cell, and a step of selecting the candidate substance by using, as an indicator, change in a protein and/or a gene of a signal transduction system of PERK and/or ATF6 in the cell. Although not limited, for example, contact with the cell is caused in the presence of or in the absence of the candidate substance, and the change in the protein and/or the gene of the signal transduction system of PERK and/or ATF6 caused by the candidate substance is measured for comparison, and thus, the candidate substance can be screened.

The eye-derived cell is not limited, and from the viewpoint of remarkably exhibiting the effects of the present invention, is preferably a cell in the sclera, and more preferably a scleral fibroblast.

Although not limited, the eye-derived cell is preferably a cell derived from an animal model where myopia has been induced. As such a myopia-induced model, a known animal model can be used.

Although not limited, examples of the myopia-induced model include an animal model caused to wear a minus lens to induce myopia, and an animal model in which myopia has been induced by administering a myopia inducing agent.

As such a minus lens, one having a power of -20 to -40 diopters (D) can be used, and the power is preferably -25 to -35 diopters (D). A method for causing the minus lens to be worn is not limited but any known methods can be employed, and for example, the minus lens is fixed in front of the eye of an animal with a fixing tool.

The period when the minus lens is worn is, for example, at least 1 week, preferably 2 weeks or more, and more preferably 3 weeks or more.

Besides, as the myopia inducing agent, any of known substances can be used, and for example, tunicamycin, thapsigargin, or the like can be used as the myopia inducing agent as evaluated in experimental examples described below. Alternatively, as the myopia inducing agent, a PERK pathway activator and an ATF6 pathway activator can be used in combination. An example of the PERK pathway activator includes CCT020312, an example of the ATF6 pathway activator includes AA147, these can be administered singly or administered as a mixture, and it is preferable that these are administered as a mixture.

Although not limited, such a myopia inducing agent can be administered in the form of an injection or eyedrops from the viewpoint of causing it to act on an eye cell of the sclera or the like, and it is preferably administered in the form of eyedrops. When tunicamycin is used in the form of eyedrops, the concentration can be, for example, 10 to 100 µg/mL, and is preferably 20 to 80 µg/mL, and more preferably 40 to 60 µg/mL.

When thapsigargin is used in the form of eyedrops, the concentration can be, for example, 1 to 100 µM, and is preferably 2 to 60 µM, and more preferably 5 to 30 µM.

As for the time period of starting myopia induction in an animal, from the viewpoint of obtaining an animal model used on the assumption of application to children, juvenile animals are preferably used. Although not limited, in using a mouse, it is preferable to start minus lens wearing in the weaning period, and the mouse is more preferably 3 weeks old. In a C57BL6 mouse or the like, the physiological axial elongation occurs from 3 weeks old to 6 weeks old. Therefore, when myopia is induced from 3 weeks old, excessive axial elongation can be accelerated in addition to the physiological axial elongation, and hence pathologic axial elongation can be thus caused. For example, when myopia induction is performed on a 3-week-old mouse, a candidate substance is preferably applied before or after the myopia induction, or during the myopia induction. When this method is employed, it is possible to evaluate influence of the candidate substance on physiological axial elongation and pathologic axial elongation. Alternatively, when white leghorn is used as the animal, from the viewpoint of obtaining an animal model used on the assumption of application to children, for example, white leghorn chick of 5 days old is preferably used.

Although not limited, in the step of contacting a candidate substance with an eye-derived cell, the candidate substance is preferably administered orally, by intraperitoneal injection, or by eyedrops, and more preferably by eyedrops. For example, when evaluation is to be performed in a cell of the sclera, the candidate substance can be contained in eyedrops to be administered.

In a step of measuring change, caused by the candidate substance, in a protein, and/or a gene of the signal transduction system of PERK and/or ATF6, any known evaluation method can be employed. Although not limited, expression of gene, or expression or secretion of a protein can be measured by known methods such as microarray, real-time PCR method, PCR method, Western blotting method, ELISA method, and immunohistochemistry.

For example, when change in a gene of the signal transduction system of PERK or ATF6 is to be measured, RNA is extracted from a cultured cell by a known RNA extraction method to be supplied to a step of quantitatively analyzing expression of mRNA.

In the step of quantitatively analyzing expression of mRNA, real-time PCR method is preferably employed although not limited. As a marker to be measured by real-time PCR method, a factor related to signal transduction described above in (Inhibitor of PERK Pathway and ATF6 Pathway) can be used as a measurement item.

Examples of a factor related to the PERK pathway include CHOP, ATF4, and GADD34.

Examples of a factor related to the ATF6 pathway include GRP78, GRP94, PDI, Cnex, HYOU, and ERdj3.

When the expression of a protein and/or a gene of the signal transduction system of PERK and/or ATF6 is inhibited by a candidate substance, the candidate substance is selected as an inhibitor of the PERK pathway and/or the ATF6 pathway, and can be used as an inhibitor of myopia progression in children.

The present invention can be practiced also in the following aspects:
Eyedrops for inhibiting myopia progression in children, comprising an inhibitor of the PERK (PKR-like endoplasmic reticulum kinase) pathway and/or the ATF6 (activating transcription factor 6) pathway as an active ingredient;
eyedrops for use in inhibition of myopia progression in children, comprising an inhibitor of the PERK (PKR-like endoplasmic reticulum kinase) pathway and/or the ATF6 (activating transcription factor 6) pathway as an active ingredient;
use of an inhibitor of the PERK (PKR-like endoplasmic reticulum kinase) pathway and/or the ATF6 (activating transcription factor 6) pathway in production of eyedrops for inhibiting myopia progression in children;
a method for inhibiting myopia progression in children, comprising causing a human to take an effective amount of an inhibitor of the PERK (PKR-like endoplasmic reticulum kinase) pathway and/or the ATF6 (activating transcription factor 6) pathway;
the eyedrops, the use, or the method described above, in which the inhibitor selectively inhibits at least the ATF6 pathway;
the eyedrops, the use, or the method described above, in which the inhibitor is an inhibitor of both the PERK pathway and the ATF6 pathway;
the eyedrops, the use, or the method described above, in which the inhibitor is at least one selected from the group consisting of phenylbutyric acid and pharmacologically acceptable salts thereof;
the eyedrops, the use, or the method described above, in which the inhibitor is sodium phenylbutyrate;
the eyedrops, the use, or the method described above, in which a content of the inhibitor is 0.01 to 5% by mass based on a total amount of the eyedrops;
the eyedrops, the use, or the method described above, in which a content of the inhibitor is 0.1 to 3% by mass based on a total amount of the eyedrops;
the eyedrops, the use, or the method described above, in which a content of the inhibitor is 0.2 to 2% by mass based on a total amount of the eyedrops;
the eyedrops, the use, or the method described above, in which the inhibition of myopia progression in children does not inhibit physiological axial elongation;
the eyedrops, the use, or the method described above, in which the inhibition of myopia progression in children inhibits pathologic axial elongation;
the eyedrops, the use, or the method described above, in which the children include a child of 7 years old or older and younger than 15 years old;
the eyedrops, the use, or the method described above, in which the eyedrops are aqueous eyedrops;
the eyedrops, the use, or the method described above, in which the eyedrops are used to be eye-dropped once or twice a day;
the eyedrops, the use, or the method described above, in which the eyedrops are used to be eye-dropped once or twice a day;
the eyedrops, the use, or the method described above, in which the eyedrops are used before a nap or before bedtime;
a screening method for an inhibitor of myopia progression in children, comprising a step of contacting a candidate substance with an eye-derived cell, and a step of selecting the candidate substance by using, as an indicator, change in a protein and/or a gene of the signal transduction system of PERK and/or ATF6 in the cell;
the screening method described above, in which the eye-derived cell is a cell derived from an animal model in which myopia has been induced;
the screening method described above, in which the animal model is an animal model in which myopia has been induced by causing a minus lens to be worn, or an animal model in which myopia has been induced by administering a myopia inducing agent;
the screening method described above, in which the minus lens is a lens having a power of -20 to -40 diopters (D);
the screening method described above, in which a period when the minus lens is worn is at least 1 week;
the screening method described above, in which the myopia inducing agent contains tunicamycin, and/or thapsigargin;
the screening method described above, in which a concentration of the tunicamycin administered by eyedrop is 10 to 100 µg/M;
the screening method described above, in which a concentration of the thapsigargin administered by eyedrop is 1 to 100 µM;
the screening method described above, in which the animal model starts to wear the minus lens in the weaning period;
the screening method described above, in which the step of contacting a candidate substance with an eye-derived cell includes administering the candidate substance orally, by intraperitoneal injection, or by eyedrop administration;
the screening method described above, comprising selecting the candidate substance as an inhibitor of myopia progression in children when expression of the protein and/or the gene of the signal transduction system of PERK and/or ATF6 is inhibited by the candidate substance; and
the screening method described above, comprising selecting the candidate substance as an inhibitor of myopia progression in children when expression of the protein and/or the gene of the signal transduction system of PERK and ATF6 is inhibited by the candidate substance.

### Examples

Now, the present invention will be specifically described by way of experimental results.

### [Experimental Method]

All animal experiments performed in the present experiment were approved by Institutional Animal Experiment Committee at KEIO University, and were performed in compliance with Guideline for Care and Use of Laboratory Animals of KEIO University, ARVO Statement for the Use of Animals in Ophthalmic and Vision Research, and Animal Research: Reporting of *in vivo* Experiments (ARRIVE) Guideline.

### (Features of Myopia Induced Juvenile Mouse)

As described in Non Patent Literature 3, eyes of a human are hyperopic immediately after birth, and thereafter, the eye axes elongate (namely, become myopic), and the eyes become emmetropic in a school period (about 8 years old). Besides, as described in Non Patent Literature 6, also in a period of 3 to 6 weeks old of a mouse (C57BL6), the eye axis elongates along with growth. Accordingly, this myopia induced juvenile mouse corresponds, in terms of movement of myopia progression, to a human of about 8 years old, which substantially corresponds to infants/children (of the school age). When this animal model is used, the mechanism of myopia progression in human children can be clarified, and a therapeutic agent of myopia progression in children can be screened.

The mechanism for inducing axial myopia by causing a minus lens to be worn is schematically illustrated in Figure 1(a). Emmetropia refers to a state where an image is clearly seen because parallel rays entering the eyes is focused on the retina. On the other hand, axial myopia refers to a state where an image cannot be clearly seen because parallel rays entering the eyes are focused in front of the retina due to elongated eye axes. Eyes of animals, including a human, become large with growth. When a juvenile mouse is caused to wear a minus lens, the eye axis elongates to a focusing position obtained in wearing the minus lens, namely, up to a state where an image is clearly seen in wearing the minus lens. As a result, the eye axis elongates, and thus, an eye state similar to the state of an axial myopic eye can be created.

### (Production of Myopia-induced Juvenile Mouse)

Specifically, a myopia-induced juvenile mouse is produced as follows. It is noted that myopia induction, and measurement of an axial length and refraction were performed by methods similar to those described in Non Patent Literatures 4 and 5. First, a male C57BL6J mouse was put in a standard transparent cage under a 12/12 h light/dark cycle in a temperature controlled clean room. The animal was allowed to free access to standard feed and autoclaved tap water. A 3-week-old mouse immediately after weaning was anesthetized with a mixed anesthetic of three anesthetics, that is, Domitor (Nippon Zenyaku Kogyo Co., Ltd.), Butorphanol (Meiji Seika Pharma Co., Ltd.), and Midazolam (Sandoz K.K.), and the skull was exposed with scissors. As illustrated in Figure 1(b), a post was provided to stand on the skull, and fixed with dental cements (Super-Bond, Sun Medical Company, Ltd.). The post was provided with a screw thread so that an adjustor described below could be fixed thereon with a nut.

For inducing myopia, a minus lens having a power of -30 diopters (D) (Rainbow Contact, RAINBOW OPTICAL LABORATORY CO., LTD.) was worn on the right eye (myopia-induced eye), and a lens having a power of 0 D or only a frame was worn as a control on the left eye (control eye). A protector in a shape projecting sideways was attached to a frame portion below the lens so that the mouse did not damage the lens with the forelegs or the like when the lens was worn on the mouse. Owing to the protector, the mouse could not touch the lens, and hence the lens was not damaged. The protector used here was attached to be integral with the frame portion, but the protector needs not be integral with the lens as long as the lens is not damaged by the behavior of the mouse. For example, the protector may be in a shape similar to an Elizabeth collar worn by an injured animal.

In the frame portion above the lens, the adjustor for adjusting the width and the angle of the worn lens in accordance with the growth of the mouse was attached. The adjuster was in a bent dogleg shape, one end thereof was attached to the lens, and the other end thereof was provided with a slotted hole so that it could be attached to the post provided to stand on the head. When the post was inserted through the slotted hole to be screwed with a nut, the adjustor could be fixed to be close contact with the skin without pressing the peripheries of the eye of the mouse. Owing to the adjustment mechanism composed of the post, the nut and the adjustor, the width and the angle could be adjusted in accordance with the growth of the mouse, so that the lens could be disposed in a position corresponding to the eye of the mouse. Besides, since the lens was removable, change over time in the axial length and the refraction value could be measured.

### (Measurement of Axial Elongation and Refraction)

After causing the frame alone to be worn on the control eye, and a 30 D lens to be worn on the myopia-induced eye for 3 weeks, the refraction value and the axial length were measured to obtain differences caused through the wear. The refraction value was measured with a refractometer (Infrared Photorefractor for Mice, produced by Professor Schaeffel, Tubingen University), and the axial length was measured with SD-OCT (Spectral-domain OCT, spectral-domain optical coherence tomography, Envisu R4310, Bioptigen Inc.).

### (Preparation of Test Drug)

Sodium phenylbutyrate (Cayman Chemical, MI, USA) and tauroursodeoxycholic acid (Sigma Aldrich, Tokyo, Japan) were dissolved in PBS. STF080310 (Selleck Biotech, Tokyo, Japan) or 4µ8C (Selleck Biotech) of an IRE1 inhibitor, GSK2656157 (Selleck Biotech) or GSK2606414 (Selleck Biotech) of a PERK inhibitor, and Nelfinavir Mesylate hydrate (Tokyo Chemical Industry Co., Ltd.) or Ceapin-A7 (Sigma Aldrich) of an ATF6 inhibitor were dissolved in DMSO, and the resultant was diluted to 1: 1000 with PBS to be used in an eyedrop test.

### (Eyedrop)

During the myopia induction, a solution of 0.2% or 2% sodium phenylbutyrate in PBS (4-PBA), 60 µM STF080312 (STF), 100 µM 4µ8C (4µ8C), 100 µM GSK2656157 (GSK), 100 µM GSK2606414 (GSK2606414), 100 µM nelfinavir mesylate hydrate (NFV), or 100 µM Ceapin-A7 (Ceapin) was eye-dropped to both eyes once a day every day in the evening for 10 days.

### (Intraperitoneal Injection)

A solution of sodium phenylbutyrate in PBS (4-PBA; 200 mg/kg) or tauroursodeoxycholic acid (TUDCA; 100 mg/kg) was intraperitoneally injected (i.p.) every day during the myopia induction period.

### (Concentration Measurement and Analysis by Western Blotting Method)

A protein (10 µg/well) of the sclera was separated by SDS-PAGE, transferred onto a PVDF membrane (Merck Millipore, MA, USA), blocked with Blocking One (Nacalai Tesque, Tokyo), and incubated together with anti-ATF6 (BioAcademia), phosphorylation-IRE1 (Ser724, Abeam, Cambridge, UL), IRE1, phosphorylation-eIF2a, eIF2a, and β-actin (Cell Signaling Technologies Japan, Tokyo, Japan) antibodies at 4°C overnight. The resultant membrane was incubated with an appropriate HRP-bound secondary antibody, and the resultant was visualized with EzWestLumi plus (ATTA, Tokyo, Japan). SDS-PAGE was performed in a 10% acrylamide gel with a protein size marker (Magic Mark XP Western Protein Standard, ThermoFisher Scientific). The concentration measurement and analysis was performed using Image J software.

### (Quantitative PCR)

Quantitative real-time PCR was performed using StepOnePlus Realtime PCR system with PowerUp SYBR Green Master Mix (Applied Biosystems, CA, USA). Expression level was standardized using β-actin.

### (Statistical Analysis)

Data obtained in the experiment were all indicated as an average ± standard deviation. A difference between groups was analyzed by Student's t-test, one-way analysis of variance, or using a generalized estimating equation. When a significant difference was obtained by ANOVA, Tukey HSD was next performed to determine significance of a difference between averages. A p value of less than 0.05 indicates a statistically significant difference.

### [Experimental Results]

### <Test Example 1 Change in Expression of Endoplasmic Reticulum Stress Response Genes in Sclera of Myopia Induced Juvenile Mouse>

In order to evaluate involvement, in myopia progression in children, of the PERK pathway, the ATF6 pathway, and the IRE1 pathway, that is, the endoplasmic reticulum stress response genes, the expression of the genes in the sclera of a mouse simulating the myopia progression in children was evaluated in accordance with the description given above in "Experimental Method".

Figure 2 illustrates axial elongation (a) and refraction change (b) caused after myopia induction for 3 weeks, and Figure 3 illustrates change in the expression of the genes in the sclera at that time point. Besides, the pathways of the endoplasmic reticulum stress response genes are illustrated in Figure 4. As a result, in the sclera of the myopia induced juvenile mouse, expression of genes disposed downstream of PERK, ATF6, and IRE1, that is, the principal endoplasmic reticulum stress response genes (Figure 4), was significantly increased (Figure 3).

It was confirmed based on these results that the expression of the genes of the PERK pathway, the ATF6 pathway, and the IRE1 pathway increases in accordance with myopia progression in children.

### <Test Example 2 Myopia Progression Inhibition by Various Inhibitors of Endoplasmic Reticulum Stress Response Genes>

It was suggested in Test Example 1 that PERK, ATF6, and IRE1 are involved in myopia progression in children, and therefore, in this Test Example 2, it was evaluated how the phenotype of myopia progression was affected by known inhibitors of these genes. It is noted that GSK2656157 (GSK) and GSK2606414 were used as the PERK inhibitor, Nelfinavir Mesylate hydrate (NFV) and Ceapin-A7 were used as the ATF6 inhibitor, and STF080312 (STF) and 4µ8C were used as the IRE1 inhibitor (Figure 5).

Figures 6-a and 6-b illustrate axial elongation (a) and refraction change (b) obtained after eyedrop administration of 60 µM STF, 100 µM GSK, or 100 µM NFV once a day for 10 days during the myopia induction period of the mouse. Figures 6-c and 6-d illustrate axial elongation (a) and refraction change (b) obtained after similarly eyedrop administration of a combination of these inhibitors (STF + GSK: S + G, GSK + NFV: G + N, NFV + STF: N + S, or STF + GSK + NTF: S + G + N). Besides, Figure 7 illustrates axial elongation (a) and refraction change (b) obtained after similarly eyedrop administration of 100 µM GSK2606414, Ceapin-A7, or 4µ8C.

As a result, in the sclera of the myopia induced juvenile mouse, when each of the inhibitors of the principal endoplasmic reticulum stress response genes of PERK, ATF6 and IRE1 was singly eye-dropped, neither inhibition of axial elongation nor inhibition of myopic refraction was found in the sclera of the myopia-induced juvenile mouse contrary to expectation (Figures 6-a and 6-b). Instead, when each of the inhibitors excluding STF was singly eye-dropped, the eye axis of the control eye in which myopia had not been induced significantly elongated to cause myopic refraction as compared with that caused by eyedrop administration of DMSO. Besides, when a combination of these was eye-dropped, only when at least two of the PERK inhibitors and the ATF6 inhibitors (G + N, and S + G + N) were included in the combination, axial elongation was significantly inhibited, myopic refraction was inhibited, and the axial elongation caused in the control eye, which was caused by eyedrop administration of a single one of these, was not caused (Figures 6-c and 6-d). In addition, axial elongation and refraction change caused by eyedrop administration of single one of the inhibitors GSK2606414, Ceapin-A7, and 4µ8C, which are different from those used in Figure 6, were completely the same as the results illustrated in Figure 6, and neither axial elongation nor myopic refraction was inhibited, and the eye axis of the control eye also elongated when each of those excluding 4µ8C was singly eye-dropped (Figure 7).

It was confirmed based on these results that axial elongation is inhibited and myopic refraction is inhibited when at least both of PERK and ATF6, among the principal endoplasmic reticulum stress pathway genes (PERK, ATF6, and IRE1), are inhibited. It was confirmed that pathologic axial elongation is caused beyond the axial elongation along with the growth by inhibiting PERK singly, ATF6 singly, a combination of PERK and IRE1, and a combination of ATF6 and IRE1. Based on this, it was considered that the inhibition of the PERK pathway and the ATF6 pathway is significant for myopia inhibition. Although not limited, from the viewpoint of searching an inhibitor of myopia progression in children, it was considered to be effective to search a component capable of inhibiting both the PERK pathway and the ATF6 pathway.

### <Test Example 3 Inhibition of Endoplasmic Reticulum Stress Pathway by 4-PBA and TUDCA>

It is considered, in Test Example 2, that it is effective for search of an inhibitor of myopia progression in children to search a component inhibiting both the PERK pathway and the ATF6 pathway, and therefore, in this Test Example 3, components conventionally known to inhibit axial elongation (sodium phenylbutyrate {4-PBA}, and tauroursodeoxycholic acid {TUDCA}) were evaluated for inhibition profile against the endoplasmic reticulum stress pathway.

Figure 8 illustrates change in gene expression obtained after intraperitoneal injection of 4-PBA every day through the myopia induction period. Figure 9 illustrates axial elongation (a) and refraction change (b) caused by 4-PBA administration in 1st week and 3rd week of the myopia induction. Besides, Figure 10 illustrates axial elongation (a) and refraction change (b) obtained by eyedrop administration of 0.2% or 2% 4-PBA to a mouse during myopia induction. Figure 11 illustrates axial elongation (b) and refraction change (a) obtained by intraperitoneal injection of TUDCA every day over the myopia induction period.

As a result, the expression of genes disposed downstream of the PERK pathway and the ATF6 pathway overactivated in the myopia-induced juvenile mouse (Figure 4) was significantly inhibited by eyedrop administration of 2% 4-PBA (Figure 8). Besides, the axial elongation caused by eyedrop administration of 2% 4-PBA in 1st and 3rd weeks was significantly inhibited to the same extent, and myopic refraction was also significantly inhibited to the same extent. Besides, axial elongation of the control eye in which myopia had not been simultaneously induced (physiological axial elongation) was not inhibited but pathologic axial elongation of the myopia-induced eye alone was inhibited (Figure 9). Furthermore, when the axial elongation and the refraction change were compared between those caused by 0.2% 4-PBA and by 2% 4-PBA, dose-dependent effectiveness was found (Figure 10). Similarly, also the administration of TUDCA, which is known as an inhibitor of endoplasmic reticulum stress, did not inhibit physiological axial elongation but inhibited pathologic axial elongation similarly to 4-PBA (Figure 11).

It was confirmed, based on these results, that 4-PBA and TUDCA inhibit the PERK pathway and the ATF6 pathway corresponding to the mechanism of inhibition of myopia progression in children among the endoplasmic reticulum stress pathways (Figure 12), and do not inhibit physiological axial elongation along with normal eye growth but inhibit only pathologic axial elongation. In other words, it was confirmed that a component inhibiting both PERK and ATF6, such as a combination of 4-PBA, TUDCA, a PERK inhibitor, and an ATF6 inhibitor, does not inhibit normal refraction change from hyperopia to emmetropia in children, but inhibits only myopia progression beyond it.

Based on the results of Test Examples 1 to 3, through the evaluation using myopia induced juvenile mice simulating myopia progression following emmetropization in human children, the mechanism of the pathologic axial elongation (overactivation of the PERK pathway and the ATF6 pathway caused by endoplasmic reticulum stress) was clarified, and based on this mechanism, a searching method for an inhibitor of myopia progression in children was found. In addition, it was confirmed that 4-PBA and TUDCA have, in addition to a simple effect of inhibiting axial elongation, an effect of inhibiting only pathologic axial elongation without inhibiting physiological axial elongation by inhibiting the PERK pathway and the ATF6 pathway. In other words, it was confirmed *in vivo* that an inhibitor for simultaneously inhibiting the PERK pathway and the ATF6 pathway can suitably prevent/treat myopia progression in children.

### <Test Example 4 Involvement of ATF6 Pathway in Inhibition of Myopia Progression in Children>

It was further examined to what extent the ATF6 pathway, out of both the pathways of the PERK pathway and the ATF6 pathway, was involved in the inhibition of myopia progression in children. In accordance with the description given above in [Experimental Method], concentration measurement and analysis was performed by Western blotting method to obtain the amount of activated form of ATF6 (ATF6-N) and the amount of precursor form of ATF6 (ATF6-P). The method by eyedrop administration of single one of PERK, ATF6, and IRE1 inhibitors or the method by eyedrop of a combination of these inhibitors in the sclera of a myopia-induced juvenile mouse was the same as those described in Test Example 2.

A value obtained by dividing the amount of activated form of ATF6 (ATF6-N) by the amount of precursor form of ATF6 (ATF6-P) is illustrated as an activation indicator in Figure 13.

As illustrated in Figure 13, the value obtained by dividing the amount ATF6-N of activated form of ATF6 by the amount ATF6-P of precursor form of ATF6 was significantly large in some groups. In particular, groups having a high value of the activated ATF6 when two inhibitors were used in combination accord with the groups in which pathologic axial elongation and refraction value reduction were caused as respectively illustrated in Figure 6c and Figure 6d. In other words, it is revealed that when ATF6 is inactivated in a myopia-induced eye (LIM eye), myopia is inhibited. This correlation is found through comparison between Figures 6c and 6d and Figure 13, and is summarized as shown in Table 1 below. In Table 1, "STF" or "S" indicates the IRE1 inhibitor, "GSK" or "G" indicates the PERK inhibitor, and "NFV" or "N" indicates the ATF6 inhibitor in the same manner as in Test Examples described above. The results summarized in Table 1 show that refraction value reduction associated with axial elongation is triggered by activation of the ATF6 pathway in the sclera of a myopic eye, and on the contrary, that refraction value reduction associated with axial elongation is inhibited by inactivation of the ATF6 pathway. For treatment/prevention of myopia progression in children, a drug having such pharmacological effects (4-PBA) is effective.

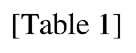

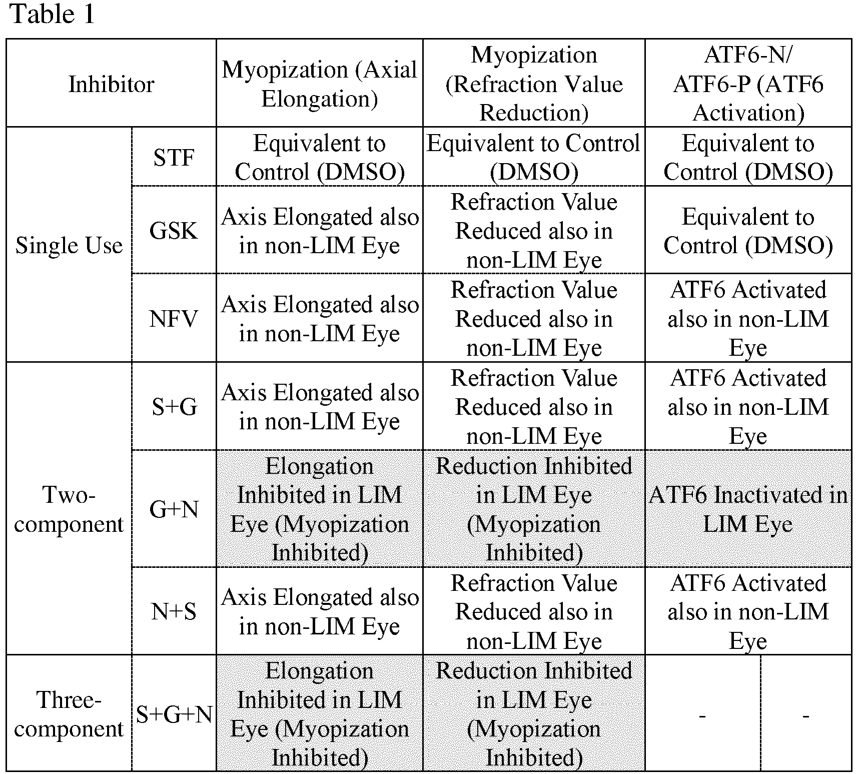

### <Test Example 5 Influence of Eyedrop Administration on Lens Thickening Caused by Myopia Induction>

It has been confirmed that the lens tends to be slightly thickened by myopia induction (LIM). It was examined whether or not a difference in the dosage form of 4-PBA affects the change in the lens. In accordance with the description given above in "Experimental Method", 4-PBA was eye-dropped or intraperitoneally administered to a myopia-induced juvenile mouse, and the thickness of the lens was measured with SD-OCT in the same manner as in the measurement of the axial length.

As illustrated in Figure 14(A), it was confirmed that the lens is thickened through myopia induction (LIM) in -30 D lens wearing group as compared with that in DMSOP eye-dropped NL (= no lens) group. Intraperitoneal administration of 4-PBA did not affect lens thickening. On the other hand, as illustrated in Figure 14(B), when 4-PBA was administered by eyedrop, lens thickening was not caused in the -30 D lens wearing group. In other words, it was revealed that eyedrop administration is favorable as the method for administering 4-PBA in terms of reachability to a target tissue.

## Claims

1. Eyedrops for inhibiting myopia progression in children, comprising an inhibitor of PERK (PKR-like endoplasmic reticulum kinase) pathway and/or ATF6 (activating transcription factor 6) pathway as an active ingredient.

2. The eyedrops according to claim 1, wherein the inhibitor is at least one selected from the group consisting of phenylbutyric acid and pharmacologically acceptable salts thereof.

3. The eyedrops according to claim 1 or 2, wherein the inhibitor is sodium phenylbutyrate.

4. The eyedrops according to any one of claims 1 to 3, wherein a content of the inhibitor is 0.01 to 5% by mass based on a total amount of the eyedrops.

5. The eyedrops according to any one of claims 1 to 4, wherein inhibition of the myopia progression in children does not inhibit physiological axial elongation.

6. The eyedrops according to any one of claims 1 to 5, wherein inhibition of the myopia progression in children inhibits pathologic axial elongation.

7. A screening method for an inhibitor of myopia progression in children, comprising a step of contacting a candidate substance with an eye-derived cell; and a step of selecting the candidate substance by using, as an indicator, change in a protein and/or a gene of a signal transduction system of PERK and/or ATF6 in the cell.
